**Europäisches Patentamt**

**European Patent Office**

**Office européen des brevets**

(19)

(11) Veröffentlichungsnummer: **0 327 939**

**A2**

(12) # EUROPÄISCHE PATENTANMELDUNG

(21) Anmeldenummer: **89101690.9**

(22) Anmeldetag: **01.02.89**

(51) Int. Cl.⁴: **C12P 21/00 , C12P 21/02 , C12N 15/00 , G01N 33/68**

Patentansprüche für folgende Vertragsstaaten:
ES + GR.

(30) Priorität: **11.02.88 DE 3804148**

(43) Veröffentlichungstag der Anmeldung:
**16.08.89 Patentblatt 89/33**

(84) Benannte Vertragsstaaten:
**AT BE CH DE ES FR GB GR IT LI LU NL SE**

(71) Anmelder: **Plückthun, Andreas, Dr.**
**Veit-Lung-Strasse 6**
**D-8033 Planegg(DE)**

Anmelder: **Glockshuber, Rudolf**
**Plassenburger Strasse 12**
**D-8000 München 90(DE)**

Anmelder: **Ellinger, Sylvia**
**Kuttlerstrasse 13**
**D-8520 Erlangen(DE)**

Anmelder: **Jahn, Gerhard, Prof. Dr.**
**Effeltricher Strasse 11**
**D-8524 Neunkirchen(DE)**

(72) Erfinder: **Plückthun, Andreas, Dr.**
**Veit-Lung-Strasse 6**
**D-8033 Planegg(DE)**
Erfinder: **Glockshuber, Rudolf**
**Plassenburger Strasse 12**
**D-8000 München 90(DE)**
Erfinder: **Ellinger, Sylvia**
**Kuttlerstrasse 13**
**D-8520 Erlangen(DE)**
Erfinder: **Jahn, Gerhard, Prof. Dr.**
**Effeltricher Strasse 11**
**D-8524 Neunkirchen(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Verfahren zur Spaltung von Fusionsproteinen.**

(57) Durch Einschieben von kurzen Homoglycin-Regionen nach und gegebenenfalls vor die durch eine Aminosäure wie Lys verlängerte Erkennungssequenz für den Blutkoagulationsfaktor Xa wird die Spezifität dieses Enzyms stark erhöht. Man erhält so unter Einsatz geringerer Mengen von Faktor Xa und in kürzerer Zeit eine komplette, hochspezifische Spaltung, wobei neue Spaltstücke gebildet werden. Man gewinnt so vorteilhaft Antigene, die zur Herstellung der entsprechenden Antikörper oder unmittelbar für Diagnostika geeignet sind.

## Verfahren zur Spaltung von Fusionsproteinen

Aus der europäischen Patentanmeldung mit der Veröffentlichungsnummer (EP-A) 0 161 937 ist es bekannt, in Gene für Fusionsproteine eine Spaltstelle für den Blut-Koagulationsfaktor Xa einzubauen. Dieser Faktor "erkennt" die Tetrapeptidsequenz Ile-Glu-Gly-Arg und spaltet sie C-ständig am Arg. Wie aus der genannten EP-A jedoch hervorgeht, werden auch andere Tetrapeptid-Sequenzen erkannt, so daß in der Praxis neben der erwünschten Spaltung noch weitere Spaltungen der Polypeptidkette auftreten. Dies führt zu einer verminderten Ausbeute des gewünschten Proteins und zum Auftreten von mehr Bruchstücken, wodurch das Reinigungsverfahren aufwendiger wird, was seinerseits zu einer weiteren Verminderung der Ausbeute beiträgt. Außerdem benötigt man für schwerlösliche Fusionsproteine, insbesondere solche mit einem $\beta$-Galaktosidase-Anteil, erhöhte Mengen an Faktor Xa und teilweise erheblich verlängerte Reaktionszeiten.

Erfindungsgemäß werden diese Nachteile überwunden, indem man nach und/oder vor die Kodons der für Faktor Xa spezifischen Aminosäuresequenz sowie dem Kodon für eine weitere, von Prolin verschiedene Aminosäure kurze Sequenzen aus für Glycin kodierende Tripletts setzt. Durch diese "Einrahmung" wird überraschenderweise die Spaltbarkeit durch den Faktor Xa an der gewünschten Stelle im Expressionsprodukt signifikant erhöht.

Die Erfindung bezieht sich somit auf neue Gene für Fusionsproteine mit einer Spaltstelle für den Faktor Xa, die durch eine oder zwei kurze Homo-Gly-Sequenz(en) sowie gegebenenfalls eine zusätzliche Aminosäure von den angrenzenden Proteinregionen abgehoben sind. Die Erfindung bezieht sich weiterhin auf die entsprechenden Fusionsproteine, auf Verfahren zu deren Spaltung mit Faktor Xa bzw. auf die Verwendung dieser Fusionsproteine zur Herstellung der Komponenten. In diesen Komponenten sind die aus der Spaltung hervorgehenden Enden der Proteinkette modifiziert. Die Erfindung bezieht sich deshalb vor allem auf solche neuen Spaltprodukte, die unmittelbar - also ohne Veränderung dieser Enden - einsetzbar sind, beispielsweise in diagnostischen Verfahren und Mitteln, die Antikörper gegen die Spaltprodukte verwenden. Weitere Aspekte der Erfindung sind in den Patentansprüchen definiert.

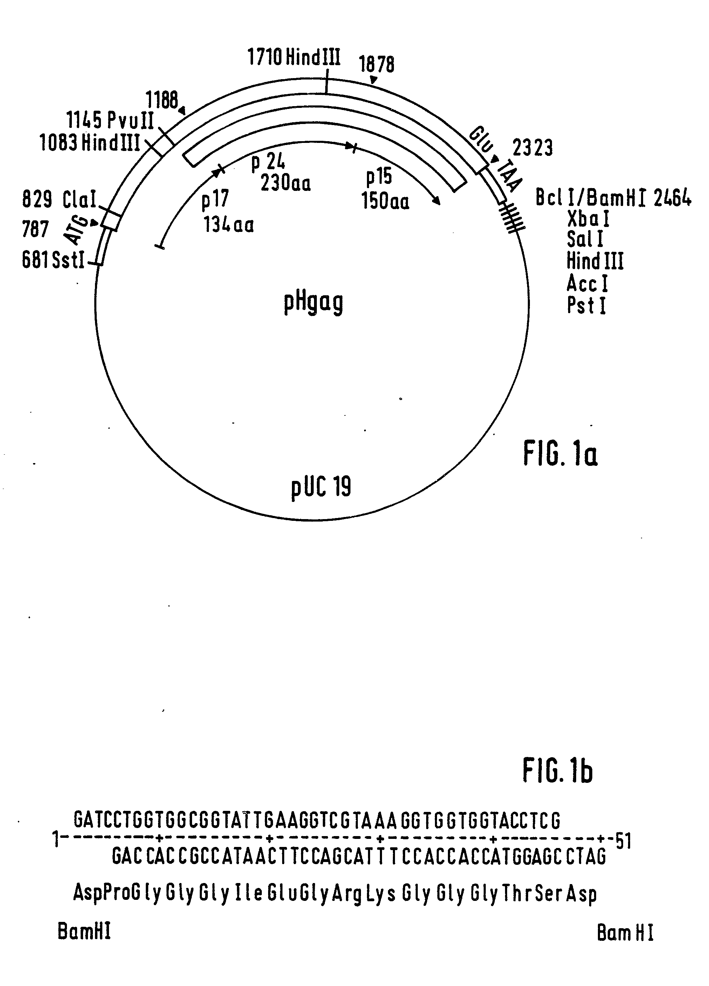

Die Fig. 1a zeigt den Vektor pH gag, der für ein erfindungsgemäßes Fusionsprotein kodiert, und die Fig. 1b eine DNA-Sequenz, die für eine charakteristische Spaltstelle kodiert.

Die neuen Gene sind gekennzeichnet durch eine eingeschobene DNA-Sequenz, die für ein Protein der Formel

$(Gly)_x$-X-Y-Gly-Arg-Z-$(Gly)_y$

kodiert, in der X für Ile, Leu, Pro oder Ala, Y für Glu, Asp, Gln oder Asn, Z für eine genetisch kodierbare Aminosäure mit Ausnahme von Pro, x und y unabhängig voneinander für ganze Zahlen bis 10, vorzugsweise 2 bis 6, insbesondere 3 oder 4, stehen, wobei x und y nicht beide Null sind. X bedeutet vorzugsweise Ile, Y vorzugsweise Glu oder Asp, insbesondere Glu und Z vorzugsweise Arg, Asp oder Glu und insbesondere Lys. Wenn Z Gly und y von Null verschieden ist, wird Z also Bestandteil der Homo-Gly-Sequenz.

Bei der Diskussion der folgenden bevorzugten Ausgestaltungen wird davon ausgegangen, daß x und y die bevorzugte Bedeutung von mindestens 2 haben. Damit sollen jedoch Ausführungsformen mit x oder y = 0 oder 1 nicht ausgeschlossen sein.

Die besonders bevorzugte Pentapeptidsequenz zwischen den beiden Homoglycin-Regionen wird vorteilhaft durch die DNA-Sequenzen (kodierender Strang) ATC GAG GGT AGG AAA oder ATT GAA GGT CGT AAA kodiert.

Die Homoglycin-Regionen werden vorteilhaft durch GGT oder GGC kodiert.

Bei der Spaltung der erfindungsgemäßen Fusionsproteine werden als Spaltprodukte neue Proteine erhalten, von denen das eine C-terminal durch die Homoglycinregion und die Aminosäurefolge X-Y-Gly-Arg charakterisiert ist, während das zweite Protein durch das N-terminale Z, gefolgt von der Homoglycin-Region, gekennzeichnet ist.

Die Erfindung eignet sich besonders zur Herstellung von immunogenen Proteinen, bei denen die genannten charakterisierenden Endgruppen, insbesondere die Oligoglycin-Gruppe, nicht stören. Man kann so erfindungsgemäß beispielsweise virale Proteine herstellen, die als Immunogen gegenüber den üblicherweise verwendeten Fusionsproteinen mit bakteriellem Anteil den Vorteil aufweisen, daß sie nicht die bakterientypischen antigenen Determinanten enthalten. Es kommt also damit nicht zu einer Kreuzreaktion mit Antikörpern auf derartige bakterielle Proteine.

Die Erfindung bezieht sich somit auch auf die Verwendung der neuen Spaltprodukte als Antigene sowie die Verwendung in Diagnostika zur Ermittlung von Antikörpern. Die Erfindung bezieht sich weiterhin auf Antikörper, die mit Hilfe dieser Antigene erhalten werden und auf die Verwendung dieser Antikörper in Diagnostika. Die entsprechenden Diagnostika auf Antikörper oder mit Antikörpern sind ebenfalls Bestandteil der Erfindung.

Die Spaltung der erfindungsgemäßen Fusionsproteine erfolgt in an sich bekannter Weise, wie sie in der EP-A 0 161 937 beschrieben und insbesondere in der nicht vorveröffentlichten deutschen Offenlegungsschrift 3 715 033 (EP-A 0 290 005) vorgeschlagen ist. Fusionsproteine mit einer Spaltstelle für Faktor Xa (Tetrapeptid) sind auch aus den EP-A 0 211 299, 0 227 938 und 0 229 998 bekannt. Erfindungsgemäß kann die Spaltung jedoch mit deutlich geringeren Mengen an Faktor Xa erfolgen, nämlich mit Mengenverhältnissen von weniger als 1:100 (Faktor Xa:Fusionsprotein), und zwar in wenigen Stunden.

Wie schon oben erwähnt wurde, ist ein weiterer Vorteil der Erfindung darin zu sehen, daß die Spaltung mit Faktor Xa in wesentlich kürzeren Zeiten erfolgt, als sie für eine komplette Spaltung der bekannten Fusionsproteine erforderlich sind. Ein typischer Spaltungsansatz dauert beispielsweise lediglich 4 bis 8 Stunden, während für ein entsprechendes Fusionsprotein ohne die Homoglycin-Regionen in dieser Zeit selbst mit zehnfach erhöhten Faktor Xa-Konzentrationen nur Spuren an Spaltprodukten auftreten.

Ein besonderer Vorteil liegt in der erleichterten Aufarbeitung der Spaltprodukte, da durch die Homoglycin-Regionen die Spezifität des Enzyms stark erhöht wird. Man erhält so nur eine Spaltung an der "Sollbruchstelle", während nach dem Stand der Technik neben dem gewünschten Produkt (bzw. den gewünschten beiden Produkten) noch eine mehr oder weniger große Anzahl von kleineren Spaltprodukten auftreten. Erfindungsgemäß können also durch geeignete Wahl des "Ballastanteils" die Eigenschaften wie Größe der beiden Spaltprodukte so eingestellt werden, daß die Auftrennung einfach wird. So ist beispielsweise die Identifizierung eines viralen Polypeptids in einer elektrophoretischen Trennung durch die geringe Zahl der Bruchstücke sehr vereinfacht.

Alle die aufgezeigten Vorteile des erfindungsgemäßen Verfahrens wirken zusammen und bewirken eine Verbesserung der Wirtschaftlichkeit in der Herstellung gentechnisch kodierbarer Polypeptide und Proteine. Diagnostische Verfahren und Mittel, bei denen solche Proteine eingesetzt werden, werden erheblich verbessert.

Die Erfindung wird im folgenden am Beispiel der HIV-Antigene p24 und p15 erläutert. Es wird hierbei von einem Fusionsprotein mit einem bakteriellen "Ballastanteil" Gebrauch gemacht. Selbstverständlich kann jedoch in an sich bekannter Weise als "Ballast-Anteil" auch das eukaryotische Protein Interleukin-2 (IL-2) dienen, wobei dann als Spaltprodukt ein Derivat des IL-2 erhalten wird (EP-A 0 227 938, 0 228 018 und 0 229 998).

## Beispiel 1

Gene für Fusionsproteine aus β-Galaktosidase und verschiedenen Proteinen des HIV-Typs I, Stamm HTLV III_B (Ratner et al., Nature 313, 277 (1985)) wurden mit an sich bekannten Methoden (Maniatis et al., Molecular Cloning, Cold Spring Harbor, 1982) wie folgt konstruiert. Als Vektor und Lieferant des β-Galaktosidase-Anteils (der ersten 375 Aminosäuren) wurde das Plasmid pBD2 (M. Bröker, Gene Anal. Tech. 3, 53 (1986)) verwendet. Für die kodierende Region des Proteins p24 der gag-Region des HTLV III_B (Ratner et al., a.a.O.) wurde das HindIII-HindIII-Fragment (630 bp, Position 1083 bis 1710 im Virus) verwendet. Es enthält einen Großteil der kodierenden Region des Proteins p24 sowie etwa 100 bp des carboxyterminalen Endes von p17. Für die kodierende Region des sich daran anschließenden Proteins p15 wurde das HindIII-BclI-Fragment (ca. 750 bp, Position 1710 bis 2464 im Virus) verwendet, welches nach Subklonieren in pUC19 um die Polylinker-Region zwischen BamHI und HindIII verlängert war und so ebenfalls als HindIII-HindIII-Fragment benutzt werden konnte. Diese Region enthält die gesamte kodierende Region von p15.

In die so erhaltenen Vektoren für die Fusionsproteine wurde in eine BamHI-Schnittstelle, die um 20 bp stromaufwärts vor der verwendeten HindIII-Stelle liegt, ein synthetisches Oligonukleotid einligiert, das die für die mit Gly eingerahmte Spaltstelle der Protease-Faktor Xa kodiert. Die Figur 1a zeigt den Vektor pH gag, die Figur 1b die in die BamHI-Schnittstelle des Polylinkers insertierte Region mit der erfindungsgemäß modifizierten Spaltstelle.

## Beispiel 2

### Isolierung der Fusionsproteine

Jedes so erhaltene Plasmid wurde in den E. coli-Stamm W3110 transformiert. In einer typischen Präparation wurden die Zellen in 400 ml LB-Medium angezogen und bei einer O.D._550 von 1,0 mit IPTG (Endkonzentration 1 mM) induziert und über Nacht unter Schütteln inkubiert. Die Zellen wurden abzentrifugiert (SORVALL Rotor GSA, 4000 rpm, 15 min, 4° C) und in 40 ml Natriumphosphat-Puffer (50 mM, pH 7,0) resuspendiert. Die Zellen wurden durch zweimalige Passage durch eine "French Press" homogenisiert.

Eine Lösung (160 ml) von 400 g Saccharose pro Liter des genannten Natriumphosphat-Puffers wurden mit dem Lysat überschichtet und zentrifugiert (SORVALL Rotor GSA, 4000 rpm, 4° C, 30 min). Der Niederschlag wurde in 1 bis 2 ml Tris-Puffer (10 mM, pH 8,3) gründlich aufgeschlämmt und unter Rühren in 40 ml einer Harnstoff-Lösung eingetropft (10 mM Tris-HCl, pH 8,3, 8 M Harnstoff). Dabei ging das Fusionsprotein in Lösung. Diese Lösung wurde erneut zentrifugiert (SORVALL-Zentrifuge, Rotor SS34, 20000 rpm, 1 Stunde, 4°C). Der Überstand wurde bei 4°C gegen Tris-Puffer (10 mM Tris-HCl, pH 8,3) dialysiert. Die Lösung blieb klar; das Fusionsprotein blieb in Lösung.

**Beispiel 3**

Das so erhaltene Fusionsprotein wurde mit Faktor Xa aus Rinderblut gespalten. Der Faktor X wurde, wie von Fujikawa et al. (Biochemistry 11 (1972) 4882) beschreiben, gereinigt und zu Faktor Xa aktiviert (Fujikawa K. et al., Biochemistry 11 (1972) 4982). Die Spaltung des Fusionsproteins mit Faktor Xa wurde bei 4°C in einem Tris-Puffer (50 mM Tris-HCl, pH 8,0, 150 mM NaCl, 1 mM CaCl₂) durchgeführt, in dem das Fusionsprotein auch löslich bleibt. Es wurden Konzentrationsverhältnisse von 1 : 100 bis 1 : 1000 (Faktor Xa: Fusionsprotein) angewandt und die Reaktion über Nacht durchgeführt.

Unter ähnlichen Bedingungen erfordern vergleichbare Fusionsproteine ohne die flankierenden Gly-Regionen oft 10mal mehr Faktor Xa, oder es ist sogar gar keine Reaktion nachweisbar.

**Beispiel 4**

**Herstellung von Diagnostika**

Durch SDS-Gel-Elektrophorese konnte gezeigt werden, daß sich die beiden Fusionsproteine in das β-Galaktosidase-Fragment ("β-gal") und ihren viralen Anteil spalten ließen. An einem Beispiel mit dem p24-Derivat ließ sich der Vorteil der beschriebenen Methode zeigen: Das Fusionsprotein wurde mit steigenden Konzentrationen von Faktor Xa gespalten und anschließend im "Immunblot" mit einem HIV-positiven Patientenserum getestet (Verdünnung 1 : 200). Es zeigte sich eine positive Reaktion mit dem ungespaltenen Fusionsprotein sowie mit dem abgespaltenen p24-Derivat, nicht jedoch mit dem bakteriellen β-gal-Anteil.

Zur Kontrolle wurde ein negatives Kaninchenserum (Verdünnung 1 : 50), das vorher mit E. coli-Proteinen immunisiert wurde, im selben Verfahren eingesetzt. Dieses reagierte mit dem ungespaltenen Fusionsprotein, nicht jedoch mit dem rein viralen Anteil. Es zeigte sich, daß das positive Signal allein auf die Reaktion gegen β-gal zurückzuführen ist. Das Kaninchenserum wurde in Ermangelung eines zur Zeit nicht zur Verfügung stehenden geeigneten menschlichen Serums eingesetzt. Es ist jedoch mit einem ähnlichen Ergebnis mit einem hochtitrigen HIV 1-negativen menschlichen Serum zu rechnen.

Die "Immunoblots" (Western-Blot) wurden im wesentlichen nach der Methode von Towbin (Proc. Natl. Acad. Sci. 76, 4350 (1979)) durchgeführt.

**Ansprüche**

1. Gen für ein Fusionsprotein mit einer Spaltungsstelle für den Blutkoagulationsfaktor Xa, kodierend für die Proteinsequenz
-(Gly)$_x$-X-Y-Gly-Arg-Z-(Gly)$_y$-
in der X Ile, Leu, Pro oder Ala, vorzugsweise Ile, Y Glu, Asp, Gln oder Asn, vorzugsweise Glu oder Gln, insbesondere Glu und Z eine genetisch kodierbare Aminosäure mit Ausnahme von Pro bedeutet, vorzugsweise Arg, Asp, Glu oder Lys, x und y ganze Zahlen bis 10 bedeuten, aber x und y nicht beide Null sind, vorzugsweise, unabhängig voneinander, Zahlen von 2 bis 6, insbesondere 3 oder 4 bedeuten.

2. Fusionsproteine, gekennzeichnet durch eine Aminosäuresequenz der Formel
-(Gly)$_x$-X-Y-Gly-Arg-Z-(Gly)$_y$-
in der X Ile, Leu, Pro oder Ala, vorzugsweise Ile, Y Glu, Asp, Gln oder Asn, vorzugsweise Glu oder Gln, insbesondere Glu und Z eine genetisch kodierbare Aminosäure mit Ausnahme von Pro bedeutet, vorzugsweise Arg, Asp, Glu oder Lys, x und y ganze Zahlen bis 10 bedeuten, aber x und y nicht beide Null sind, vorzugsweise, unabhängig voneinander, Zahlen von 2 bis 6, insbesondere 3 oder 4 bedeuten.

3. Verfahren zur Herstellung von genetisch kodierbaren Proteinen, von denen eines C-terminal die Aminosäuresequenz
(Gly)$_x$-X-Y-Gly-Arg
und das andere N-terminal die Aminosäuresequenz Z-(Gly)$_y$ aufweist, wobei X, Y, Z, x und y die in Anspruch 1 genannten Bedeutungen haben, dadurch gekennzeichnet, daß man ein Fusionsprotein nach Anspruch 2 mit Faktor Xa spaltet.

4. Protein aus genetisch kodierbaren Aminosäuren, gekennzeichnet durch eine C-terminale Aminosäuresequenz der Formel
(Gly)$_x'$-X-Y-Gly-Arg,
wobei X und Y die in Anspruch 1 genannten Bedeutungen haben und x' eine ganze Zahl von 1 bis 10 bedeutet.

5. Protein aus genetisch kodierbaren Aminosäuren, gekennzeichnet durch eine N-terminale Aminosäuresequenz der Formel
Z-(Gly)$_y'$

wobei y′ eine ganze Zahl von 1 bis 10 und Z eine genetisch kodierbare Aminosäure mit Ausnahme von Pro bedeutet.

6. Verwendung der Proteine nach Anspruch 4 oder 5 zur Herstellung von poly- oder monoklonalen Antikörpern oder in Diagnostika zur Ermittlung von Antikörpern.

7. Poly- oder monoklonale Antikörper gegen Proteine nach Anspruch 4 oder 5.

8. Diagnostika, gekennzeichnet durch einen Gehalt an einem Antikörper nach Anspruch 7 oder an einem Protein nach Anspruch 4 und/oder 5.

9. Verfahren zur Feststellung von Antikörpern, dadurch gekennzeichnet, daß man Körperflüssigkeiten mit einem Antigen nach Anspruch 4 oder 5 in Kontakt bringt.

10. Verfahren zur Feststellung von Antigenen, dadurch gekennzeichnet, daß man Körperflüssigkeiten mit einem Antikörper nach Anspruch 7 in Kontakt bringt.


Patentansprüche für folgenden Vertragsstaat: GR

1. Gen für ein Fusionsprotein mit einer Spaltungsstelle für den Blutkoagulationsfaktor Xa, kodierend für die Proteinsequenz
-$(Gly)_x$-X-Y-Gly-Arg-Z-$(Gly)_y$-
in der X Ile, Leu, Pro oder Ala, vorzugsweise Ile, Y Glu, Asp, Gln oder Asn, vorzugsweise Glu oder Gln, insbesondere Glu und Z eine genetisch kodierbare Aminosäure mit Ausnahme von Pro bedeutet, vorzugsweise Arg, Asp, Glu oder Lys, x und y ganze Zahlen bis 10 bedeuten, aber x und y nicht beide Null sind, vorzugsweise, unabhängig voneinander, Zahlen von 2 bis 6, insbesondere 3 oder 4 bedeuten.

2. Fusionsproteine, gekennzeichnet durch eine Aminosäuresequenz der Formel
-$(Gly)_x$-X-Y-Gly-Arg-Z-$(Gly)_y$-
in der X Ile, Leu, Pro oder Ala, vorzugsweise Ile, Y Glu, Asp, Gln oder Asn, vorzugsweise Glu oder Gln, insbesondere Glu und Z eine genetisch kodierbare Aminosäure mit Ausnahme von Pro bedeutet, vorzugsweise Arg, Asp, Glu oder Lys, x und y ganze Zahlen bis 10 bedeuten, aber x und y nicht beide Null sind, vorzugsweise, unabhängig voneinander, Zahlen von 2 bis 6, insbesondere 3 oder 4 bedeuten.

3. Verfahren zur Herstellung von genetisch kodierbaren Proteinen, von denen eines C-terminal die Aminosäuresequenz
$(Gly)_x$-X-Y-Gly-Arg
und das andere N-terminal die Aminosäuresequenz Z-$(Gly)_y$ aufweist, wobei X, Y, Z, x und y die in Anspruch 1 genannten Bedeutungen haben, dadurch gekennzeichnet, daß man ein Fusionsprotein nach Anspruch 2 mit Faktor Xa spaltet.

4. Protein aus genetisch kodierbaren Aminosäuren, gekennzeichnet durch eine C-terminale Aminosäuresequenz der Formel
$(Gly)_x$′-X-Y-Gly-Arg,
wobei X und Y die in Anspruch 1 genannten Bedeutungen haben und x′ eine ganze Zahl von 1 bis 10 bedeutet.

5. Protein aus genetisch kodierbaren Aminosäuren, gekennzeichnet durch eine N-terminale Aminosäuresequenz der Formel
Z-$(Gly)_y$′
wobei y′ eine ganze Zahl von 1 bis 10 und Z eine genetisch kodierbare Aminosäure mit Ausnahme von Pro bedeutet.

6. Verwendung der Proteine nach Anspruch 4 oder 5 zur Herstellung von poly- oder monoklonalen Antikörpern oder in Diagnostika zur Ermittlung von Antikörpern.

7. Poly- oder monoklonale Antikörper gegen Proteine nach Anspruch 4 oder 5.

8. Verfahren zur Feststellung von Antikörpern, dadurch gekennzeichnet, daß man Körperflüssigkeiten mit einem Antigen nach Anspruch 4 oder 5 in Kontakt bringt.

9. Verfahren zur Feststellung von Antigenen, dadurch gekennzeichnet, daß man Körperflüssigkeiten mit einem Antikörper nach Anspruch 7 in Kontakt bringt.


Patentansprüche für folgenden Vertragsstaat: ES

1. Verfahren zur Herstellung von Fusionsproteinen mit einer Spaltungsstelle für den Blutkoagulationsfaktor Xa der Proteinsequenz
-$(Gly)_x$-X-Y-Gly-Arg-Z-$(Gly)_y$-
in der X Ile, Leu, Pro oder Ala, vorzugsweise Ile, Y Glu, Asp, Gln oder Asn, vorzugsweise Glu oder Gln, insbesondere Glu, und Z eine genetisch kodierbare Aminosäure mit Ausnahme von Pro bedeutet, vorzugsweise Arg, Asp, Glu oder Lys, x und y ganze Zahlen bis 10 bedeuten, aber x und y nicht beide Null sind, vorzugsweise, unabhängig voneinander, Zahlen von 2 bis 6, insbesondere 3 oder 4 bedeuten, dadurch gekennzeichnet, daß man ein Gen, das für dieses Fusionsprotein kodiert, in einer Wirtszelle zur Expression bringt.

2. Verfahren zur Herstellung von genetisch kodierbaren Proteinen, von denen eines C-terminal die Aminosäuresequenz
$(Gly)_x$-X-Y-Gly-Arg
und das andere N-terminal die Aminosäuresequenz Z-$(Gly)_y$ aufweist, wobei X, Y, Z, x und y die in Anspruch 1 genannten Bedeutungen haben, dadurch gekennzeichnet, daß man ein nach Anspruch 1 erhältliches Fusionsprotein mit Faktor Xa spaltet.

3. Verwendung der nach Anspruch 2 erhältlichen Proteine zur Herstellung von poly- oder monoklonalen Antikörpern.

4. Verwendung der nach Anspruch 2 erhältlichen Proteine in Diagnostika zur Ermittlung von Antikörpern.

FIG. 1a

FIG. 1b

```
      GATCCTGGTGGCGGTATTGAAGGTCGTAAAGGTGGTGGTACCTCG
1 ---------+---------+---------+---------+--------+-51
      GACCACCGCCATAACTTCCAGCATTTCCACCACCATGGAGCCTAG

      AspProGlyGlyGlyIleGluGlyArgLysGlyGlyGlyThrSerAsp

BamHI                                              BamHI
```